# EUROPÄISCHE PATENTSCHRIFT

(11) **EP 0 649 841 B1**
(45) Veröffentlichungstag und Bekanntmachung des Hinweises auf die Patenterteilung: **16.05.2001**
(21) Anmeldenummer: 94810597.8
(22) Anmeldetag: 13.10.1994
(51) Int. Cl.: C07D 251/24, C08K 5/3492

(54) **Verfahren zur Herstellung von hydroxyphenylsubstituierten 1,3,5-Triazinen**
Process for the preparation of hydroxyphenyl-substituted 1,3,5-triazines
Procédé pour la préparation de 1,3,5-triazines hydroxyphényle substituées

(30) Priorität: 22.10.1993 CH 319993
(43) Veröffentlichungstag der Anmeldung: 26.04.1995
(73) Patentinhaber: Ciba Specialty Chemicals Holding Inc., 4057 Basel (CH)
(72) Erfinder: Reinehr, Dieter, Dr., D-79400 Kandern (DE); Bacher, Jean-Pierre, F-68220 Buschwiller (FR)

(56) Entgegenhaltungen:
- DE-A- 1 469 811
- US-A- 3 118 887
- E. M. SMOLIN AND L. RAPOPORT 's-Triazines and derivatives' 1967 , INTERSCIENCE PUBLISHERS INC. , NEW YORK * Seite 158, die dritte Zeile von unten- Seite 159, oben ; Verbindung XX *
- A.R. KATRITZKY ET.AL.: "", COMPREHENSIVE HETEROCYCLIC CHEMISTRY, Pergamon Press, 1984, Band 3, Nr. 2B, Seiten 512 - 517
- Ber.deutsch.chem.Ges., 23, 1890, p.2937-2938

## Beschreibung

Die vorliegende Erfindung betrifft ein einfaches, einstufiges Verfahren zur Herstellung von 2-hydroxyphenylsubstituierten 1,3,5-Triazinen ausgehend von Benzamidinen und o-Hydroxycabonsäurederivaten sowie die Verwendung der nach diesem Verfahren hergestellten Verbindungen als UV-Absorber.

Die Umsetzung von Amidinen entweder mit Chlorameisensäureethylester oder mit Phosgen zu 2-Hydroxyphenyl-s-triazinen ist bekannt, z.B. durch A. Pinner, Chem. Ber. 23, 2934 (1890). Darin wird auch die Umsetzung eines Alkylsalicylats mit Benzamidin in Ethanol/Diethylether zu 2-(2-Hydroxyphenyl)-4,6-diphenyl-1,3,5-triazin beschrieben; zit. auch in E. M. Smolin et al., s-Triazines and Derivatives, 158-159 (1967). Allerdings werden mit diesem Verfahren nur mittelmäßige Ausbeuten erzielt. Abwandlungen dieses Verfahrens werden beschrieben in US-A-3118887 und A.R. Katritzky et al., Comprehensive Heterocyclic Chemistry Vol. 3(2B), S. 512-517 (1984).

Es wurde nun gefunden, 2-hydroxyphenylsubstituierte 1,3,5-Triazine in einem einstufigen Verfahren und in guten Ausbeuten mit einfachen Ausgangsverbindungen herzustellen.

Das Verfahren zur Herstellung einer 2-hydroxyphenylsubstituierten Triazinverbindung der Formel 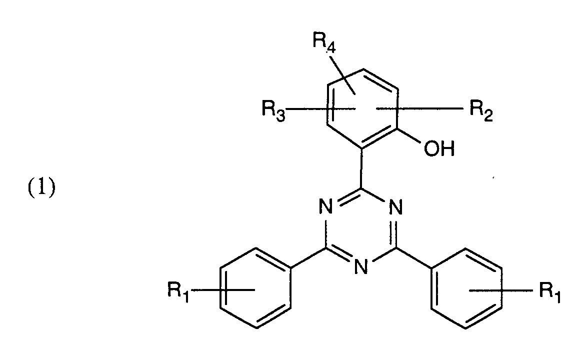 ist dadurch gekennzeichnet, dass man eine Salicylverbindung der Formel 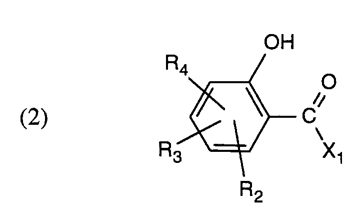 mit einer Benzamidinverbindung der Formel 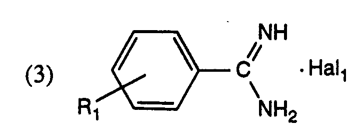 zum Triazin der Formel (1) in einem einstufigen

Verfahren in Gegenwart von Alkalialkylat, Trialkylamin, wässriger Ammoniaklösung oder Ammoniakgas als Base umsetzt, wobei
X₁ Halogen oder -OR₅,
R₁, R₂, R₃ und R₄ unabhängig voneinander Wasserstoff, Halogen, Hydroxy, C₁-C₁₈-Alkyl oder C₁-C₁₈-Alkoxy,
R₅ C₁-C₃-Alkyl und
Hal₁ Halogen bedeuten.

C₁-C₁₈-Alkyl und C₁-C₁₈-Alkoxy sind geradkettige oder verzweigte Alkyl- bzw. Alkoxyreste wie z.B. Methyl, Ethyl, n-Propyl, Isopropyl, n-Butyl, sek.Butyl, tert.Butyl, Amyl, Isoamyl oder tert.Amyl, Heptyl, Octyl, Isooctyl, Nonyl, Undecyl, Dodecyl, Tetradecyl, Pantadecyl, Hexadecyl, Heptadecyl oder Octadecyl bzw. Methoxy, Ethoxy, Propoxy, Butoxy, Pentyloxy, Hexyloxy, Heptyloxy, Octyloxy, Isooctyloxy, Nonyloxy, Undecyloxy, Dodecyloxy, Tetradecyloxy, Pentadecyloxy, Hexadecyloxy, Heptadecyloxy oder Octadecyloxy.

Halogen bedeuten Chlor, Brom oder Jod. Chlor ist bevorzugt.

Bei den Ausgangsverbindungen der Formel (2) handelt es sich um gegebenenfalls substituierte Salicylsäureester oder Salicylsäurehalogenide, wie z.B. Salicylsäuremethyl-, Salicylsäureethyl- oder Salicylsäurepropylester, Salicylsäurechlorid oder -bromid, die im Phenylrest, entsprechend der Bedeutung von R₂, R₃ und R₄ durch weitere Halogen-, Alkoxy- oder Hydroxyreste substituiert sein können.

Vorzugsweise kommen als Ausgangsverbindungen der Formel (2) Salicylsäuremethylester (X₁= -OCH₃) oder Salicylsäurechlorid (X₁=Cl) in Betracht.

Bei dem erfindungsgemässen Verfahren können die Ausgangsverbindungen der Formeln (2) und (3) in verschiedenen molaren Verhältnissen eingesetzt werden.

Vorzugsweise beträgt das Molverhältnis der Verbindung der Formel (2) zur Verbindung der Formel (3) 1:10 bis 10:1.

Verwendet man als Ausgangsverbindung der Formel (2) ein Salicylsäureester (X₁= -OCH₃), beträgt das molare Verhältnis der Verbindung der Formel (2) zur Verbindung der Formel (3) vorzugsweise 2:1 bis 1:2.

Verwendet man als Ausgangsverbindung der Formel (2) ein Salicylsäurehalogenid, beträgt das molare Verhältnis der Verbindung der Formel (2) zur Verbindung der Formel (3) 1:5 bis 1:1,5, vorzugsweise 1:3 bis 1:2.

Als Benzamidinverbindungen der Formel (3) kommen das Benzamidinhydrobromid und vorzugsweise das -chlorid in Betracht. Diese Verbindungen werden normalerweise als Festprodukte mit einem Gehalt an Aktivsubstanz von ca. 90-95% eingesetzt.

Verwendet man als Ausgangsverbindung der Formel (2) ein Salicylsäurehalogenid, wird in der Regel zur Neutralisation der bei der Reaktion entstehenden Säure mindestens die berechnete Menge einer Base zugegeben. Als Basen können wässrige Ammoniaklösung; Ammoniakgas; tert. Amine wie Pyridin oder Trialkylamine, insbesondere Triethylamin, N,N-Dimethylcyclohexylamin, N,N-Dimethylanilin; Alkalialkylate, insbesondere Natrium- und Kaliummethylat oder Kalium-tert.butylat verwendet werden.

Das erfindungsgemässe Verfahren wird in der Regel so durchgeführt, dass man die Salicyl- und die Benzamidinverbindung in einem inerten Lösungsmittel vorlegt.

Als inerte Lösungsmittel kommen dabei aliphatische Kohlenwasserstoffe und deren Mischungen, wie z.B. Cyclohexan oder aromatische Kohlenwasserstoffe wie Toluol oder Dimethylacetamid oder Mischungen dieser Lösungsmittel in Betracht.

Verwendet man als Ausgangsverbindung der Formel (2) ein Salicylsäurehalogenid (X₁=Hal), kann dem Reaktionsgemisch noch ein weiteres, in der Regel polares Lösungsmittel wie z.B. Acetonitril oder Dioxan zugegeben werden.

Die Reaktionszeiten für das erfindungsgemässe Verfahren betragen im allgemeinen 2 bis 30 Stunden. Je nachdem, ob man als Ausgangsverbindung der Formel (2) ein Salicylsäurehalogenid (X₁=Hal) oder ein Salicylsäureester (X₁= -OR₅) einsetzt, können die Reaktionszeiten variieren. Verwendet man einen Salicylsäureester, beträgt die Reaktionszeit 4 bis 30 Stunden, vorzugsweise 18 bis 22 Stunden. Verwendet man ein Salicylsäurehalogenid, sind die Reaktionszeiten etwas kürzer. Sie betragen 2 bis 20, vorzugsweise 4 bis 8 Stunden.

Die Reaktionen verlaufen in der Regel leicht exotherm ab. Allerdings sollte eine Reaktionstemperatur von 95°C nicht überschritten werden, da bei höheren Temperaturen Nebenprodukte entstehen können, wie beispielsweise Nitrilverbindungen aus den Benzamidinen. In der Praxis wird die Reaktion in einem Temperaturbereich von 60 bis 95°C, vorzugsweise 80 bis 95°C durchgeführt.

Die hydroxyphenylsubstituierten 1,3,5-Triazine fallen in der Regel als reine kristalline Verbindungen an, so dass sich eine aufwendige Umkristallisation erübrigt. Nach beendeter Reaktion wird das entstandene Produkt mit Methanol und/oder Wasser gewaschen und anschliessend auf übliche Weise getrocknet.

Mit dem vorliegenden Verfahren lassen sich hydroxyphenylsubstituierte 1,3,5-Triazine auf einfache Weise und in guten Ausbeuten herstellen.

Die nach dem erfindungsgemässen Verfahren hergestellten Triazine eignen sich als wertvolle UV-Absorber für organische Materialien, insbesondere für Polyesterfasermaterialien.

Die folgenden Beispiele veranschaulichen die Erfindung. Prozentangaben beziehen sich auf das Gewicht.

### Herstellungsbeispiele:

### Beispiel 1:

69 g (0,4 Mol) Benzamidinhydrochlorid (91%ige Ware) werden in ca. 170 ml (200g) Salicylsäuremethylester vorgelegt. Zu der Mischung werden 72 g (0,4 Mol) einer 30%igen Natriummethylatlösung und 170 ml Cyclohexan hinzugegeben. Dann wird unter Rühren auf 90°C erwärmt, wobei innerhalb von 45 Minuten ca. 130 ml einer Methanol-Cyclohexan-Mischung abdestilliert. Die Destillation wird unterbrochen und während 22 Stunden bei 90 bis 95°C gerührt, auf 5°C abgekühlt und abfiltriert. Das Nutschgut wird mit 150 ml Methanol und dann mit 2 Liter Wasser gewaschen. Nach dem Trocknen bei 120°C im Trockenschrank erhält man eine hellgelbe Verbindung der Formel 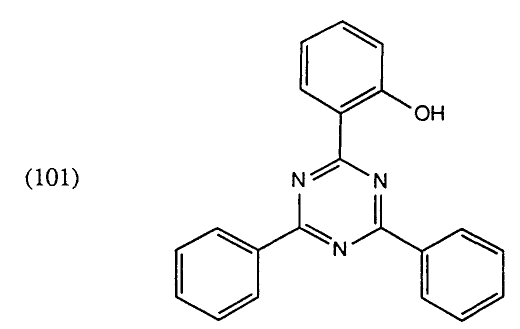 Ausbeute: 47,5 g (73% d.Th.)
Fp.: 247-248°C

### Beispiel 2:

Man verfährt wie in Beispiel 1 beschrieben mit dem Unterschied, dass man 400 g Salicylsäuremethylester (anstelle von 200 g) verwendet. Nach der Aufarbeitung erhält man 46 g der Verbindung der Formel (101), entsprechend einer Ausbeute von 70,7% d.Th.

### Beispiel 3:

Man verfährt wie in Beispiel 1 beschrieben mit dem Unterschied, dass man 42 g (0,226 Mol) 4-Methoxybenzamidinhydrochlorid, 40 g (0,22 Mol) 30%ige Natriummethylatlösung und 33 g (0,22 Mol) Salicylsäuremethylester in 100 ml Dimethylacetamid/120 ml Cyclohexan als Lösungsmittelgemisch verwendet. Nach einer Reaktionszeit von 20 Stunden bei 90 bis 95°C erhält man nach der anschliessenden Aufarbeitung ein hellgelbes Produkt der Formel 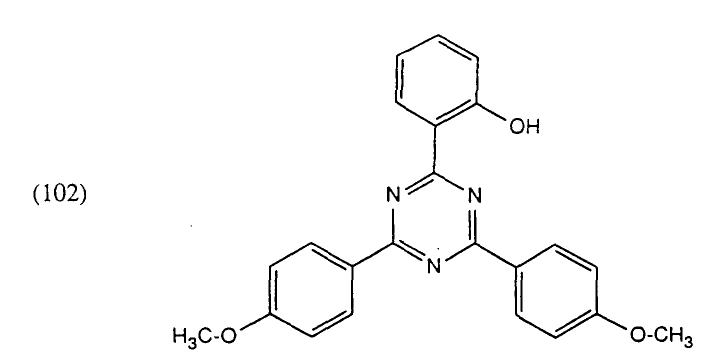 Ausbeute: 30,6 g (72,2% d.Th.)
Fp.: 205-206°C

Beispiel 4: 67,4 g (0,4 Mol) Benzamidinhydrochlorid (93%ige Ware) werden zusammen mit 200 ml Acetonitril und 61 g (0,6 Mol) Triethylamin bei Raumtemperatur während einer Stunde gerührt. Dann wird auf 0°C abgekühlt und innerhalb von 20 Minuten 31,3 g (0,2 Mol) Salicylsäurechlorid bei 0 bis 5°C zugetropft, wobei sich die Suspension intensiv gelb färbt. Es wird 22 Stunden bei Raumtemperatur gerührt, dann während 22 Stunden auf 75-80°C erwärmt, auf 60°C abgekühlt und mit 300 ml Methanol versetzt. Nach dem Abkühlen auf 5°C wird abgenutscht, mit 200 ml Methanol gewaschen und bei 110°C im Vakuumtrockenschrank getrocknet. Man erhält 49,7 g der Verbindung der Formel (101), entsprechend einer Ausbeute von 76,4 % d.Th. Fp.: 248-249°C.

### Beispiel 5:

Man verfährt wie in Beispiel 4 beschrieben mit dem Unterschied, dass man 200 ml Dioxan anstelle von 200 ml Acetonitril verwendet. Nach einer Reaktionszeit von 6,5 Stunden bei 95°C (anstelle von 22 Stunden bei 75 bis 80°C) erhält man 49 g (75,3 % d.Th.) der Verbindung der Formel (101).

### Beispiele 6 bis 13:

Entsprechend Beispiel 4 werden substituierte Salicylsäurechloride mit Benzamidinhydrochlorid bzw. mit 4-Methoxybenzamidinhydrochlorid in vergleichbaren Ausbeuten zu den entsprechenden Triazinen der unten angegebenen Strukturformel umgesetzt (Tabelle 1).

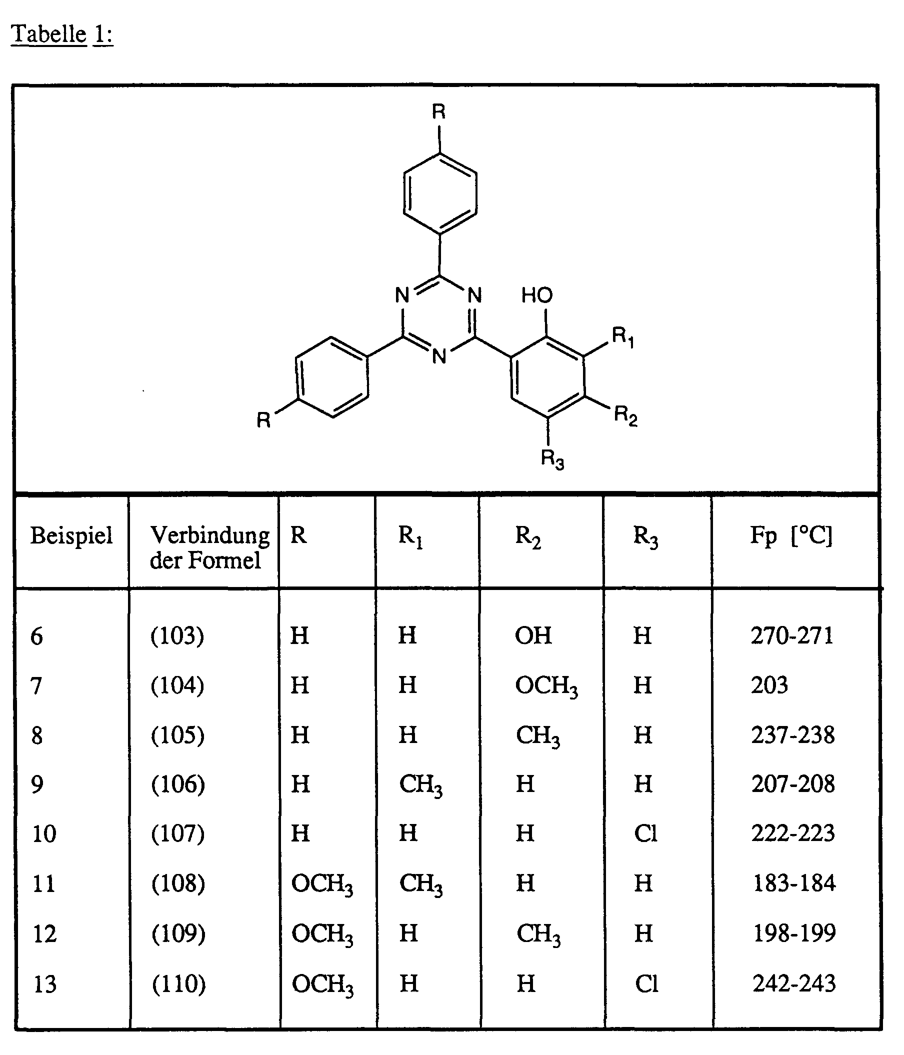

## Patentansprüche

1. Verfahren zur Herstellung einer 2-hydroxyphenylsubstituierten Triazinverbindung durch Umsetzung einer Salicylverbindung mit einem Benzamidin in einem einstufigen Verfahren, wobei die Salicylverbindung der Formel 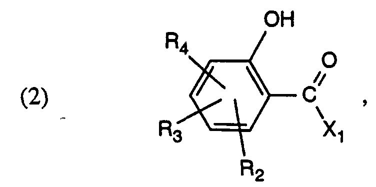 die Benzamidinverbindung der Formel (3) 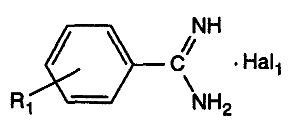 und die 2-hydroxyphenylsubstituierten Triazinverbindung der Formel 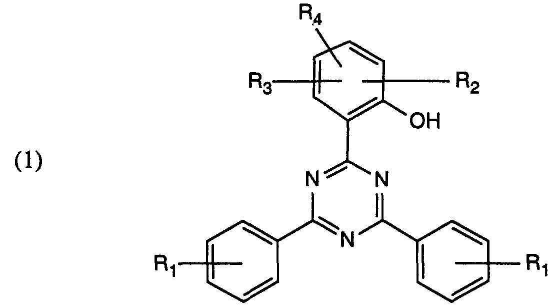 entspricht, worin
X₁ Halogen oder -OR₅,
R₁, R₂, R₃ und R₄ unabhängig voneinander, Wasserstoff, Halogen, Hydroxy, C₁-C₁₈-Alkyl oder C₁-C₁₈-Alkoxy,
R₅ C₁-C₃-Alkyl und
Hal₁ Halogen bedeuten,
dadurch gekennzeichnet, dass man die Umsetzung in Gegenwart von Alkalialkylat, Trialkylamin, wässriger Ammoniaklösung oder Ammoniakgas als Base durchführt.

2. Verfahren nach Anspruch 1, worin
X₁ Chlorid oder Methoxy bedeutet.

3. Verfahren nach Anspruch 1 oder 2, worin das Molverhältnis der Verbindungen der Formel (2) zur Verbindung der Formel (3) 1:10 bis 10:1 beträgt.

4. Verfahren nach Anspruch 3, worin das Molverhältnis der Verbindung der Formel (2) zur Verbindung der Formel (3) 2:1 bis 1:2 beträgt, wenn X₁ -OR₅ bedeutet.

5. Verfahren nach Anspruch 3, worin das Molverhältnis der Verbindung der Formel (2) zur Verbindung der Formel (3) 1:3 bis 1:2 beträgt, wenn X₁ Halogen bedeutet.

6. Verfahren nach einem der Ansprüche 1 bis 5, worin die Reaktion in Anwesenheit eines inerten Lösungsmittels durchgeführt wird.

7. Verfahren nach Anspruch 6, worin als Lösungsmittel Cyclohexan, Toluol, Dimethylacetamid oder Mischungen dieser Lösungsmittel verwendet werden.

8. Verfahren nach Anspruch 6, worin als zusätzliches Lösungsmittel Acetonitril oder Dioxan verwendet wird, wenn X₁ in Formel (2) Halogen bedeutet.

9. Verfahren nach einem der Ansprüche 1 bis 8, worin die Reaktionszeit 2 bis 30 Stunden beträgt.

10. Verfahren nach Anspruch 9, worin die Reaktionszeit 4 bis 30 Stunden beträgt, wenn X₁ in Formel (2) -OR₅ bedeutet.

11. Verfahren nach Anspruch 9, worin die Reaktionszeit 2 bis 20 Stunden beträgt, wenn X₁ in Formel (2) Halogen bedeutet.

12. Verfahren nach einem der Ansprüche 1 bis 11, worin die Reaktionstemperatur 80 bis 95°C beträgt.

13. Verfahren nach einem der Ansprüche 1 bis 12, worin als Base Alkalialkylat oder Trialkylamin verwendet wird.

## Claims

1. A process for the preparation of a 2-hydroxyphenyl-substituted triazine compound by reacting a salicyl compound with a benzamide in a one-step process, where the salicyl compound is of formula 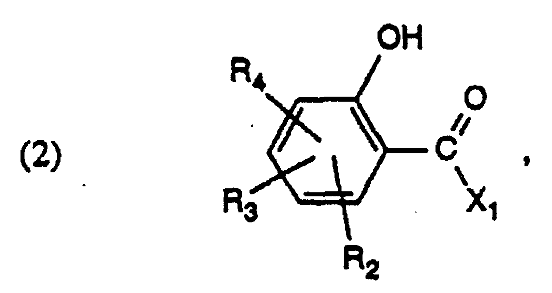 the benzamide compound is of the formula 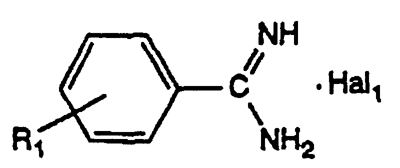 and the 2-hydroxyphenyl-substituted triazine compound is of formula 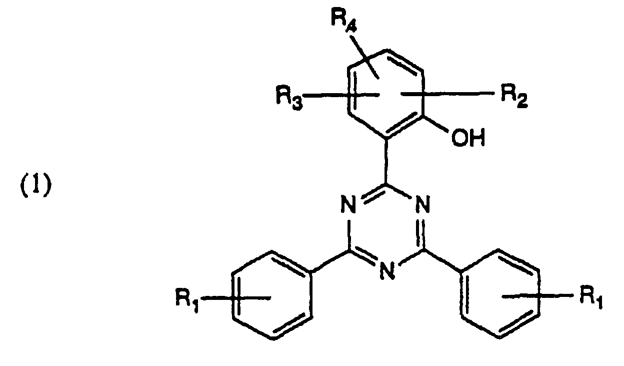 in which
X₁ is halogen or -OR₅,
R₁, R₂, R₃ and R₄ are each independently of one another hydrogen, halogen, hydroxyl, C₁-C₁₈alkyl or C₁-C₁₈alkoxy,
R₅ is C₁-C₃alkyl, and
Hal₁ is halogen,
which comprises conducting the reaction in the presence or alkali metal alkylate, trialkylamine, aqueous ammonia solution or ammonia gas as base.

2. A process according to claim 1, wherein X₁ is chloride or methoxy.

3. A process according to claim 1 or 2, wherein the molar ratio of the compounds of formula (2) to the compound of formula (3) is 1:10 to 10:1.

4. A process according to claim 3, wherein the molar ratio of the compound of formula (2) to the compound of formula (3) is 2:1 to 1:2 when X₁ is -OR₅.

5. A process according to claim 3, wherein the molar ratio of the compound of formula (2) to the compound of formula (3) is 1:3 to 1:2 when X₁ is halogen.

6. A process according to one of claims 1 to 5, wherein the reaction is carried out in the presence of an inert solvent.

7. A process according to claim 6, wherein as solvent cyclohexane, toluene, dimethylacetamide or mixtures of these solvents is/are used.

8. A process according to claim 6, wherein acetonitrile or dioxane is used as additional solvent when X₁ in formula (2) is halogen.

9. A process according to one of claims 1 to 8, wherein the reaction time is from 2 to 30 hours.

10. A process according to claim 9, wherein the reaction time is from 4 to 30 hours when X₁ in formula (2) is -OR₅.

11. A process according to claim 9, wherein the reaction time is from 2 to 20 hours when X₁ in formula (2) is halogen.

12. A process according to one of claims 1 to 11, wherein the reaction temperature is from 80 to 95°C.

13. A process according to one of claims 1 to 12, wherein alkali metal alkylate or trialkylamine is used as base.

## Revendications

1. Procédé pour la préparation d'un composé triazine 2-hydroxyphényle substitué par réaction d'un composé salicylique avec une benzamidine dans un procédé en une seule étape, dans lequel le composé salicylique correspond à la formule 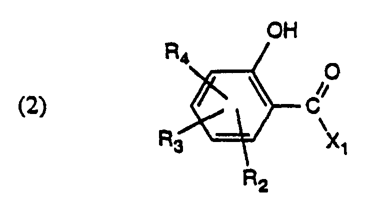 le composé benzamidine à la formule (3) 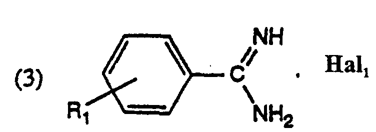 et le composé triazine 2-hydroxyphényle substitué à la formule 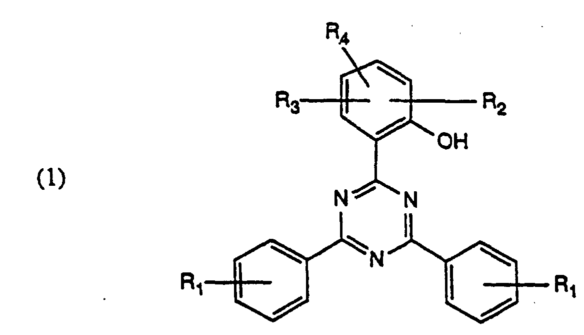 dans lesquelles
X₁ représente un atome d'halogène ou -OR₅,
R₁, R₂, R₃ et R₄ représentent indépendamment les uns des autres un atome d'hydrogène, d'halogène, un groupe hydroxy, alkyle en C₁ à C₁₈ ou alcoxy en C₁ à C₁₈,
R₅ représente un groupe alkyle en C₁ à C₃ et
Hall représente un atome d'halogène,
caractérisé en ce que, l'on réalise la réaction en présence d'alkylate de métal alcalin, de trialkylamine, de solution aqueuse d'ammoniac ou d'ammoniac gazeux comme base.

2. Procédé selon la revendication 1, dans lequel X₁ représente un chlorure ou un méthoxy.

3. Procédé selon la revendication 1 ou 2, dans lequel le rapport molaire des composés de formule (2) par rapport au composé de formule (3) s'élève de 1 : 10 à 10 : 1.

4. Procédé selon la revendication 3, dans lequel le rapport molaire du composé de formule (2) par rapport au composé de formule (3) s'élève de 2 : 1 à 1 : 2, lorsque X₁ représente -OR₅.

5. Procédé selon la revendication 3, dans lequel le rapport molaire du composé de formule (2) par rapport au composé de formule (3) s'élève de 1 : 3 à 1 : 2, lorsque X₁ représente un atome d'halogène.

6. Procédé selon l'une des revendications 1 à 5, dans lequel la réaction est réalisée en présence d'un solvant inerte.

7. Procédé selon la revendication 6, dans lequel on utilise comme solvant le cyclohexane, le toluène, le diméthylacétamide ou des mélanges de ces solvants.

8. Procédé selon la revendication 6, dans lequel on utilise comme solvant supplémentaire l'acétonitrile ou le dioxane, lorsque X₁ représente un atome d'halogène dans la formule (2).

9. Procédé selon l'une des revendications 1 à 8, dans lequel le temps de réaction se monte à entre 2 et 30 heures.

10. Procédé selon la revendication 9, dans lequel le temps de réaction se monte à entre 4 et 30 heures, lorsque X₁ représente -OR₅ dans la formule (2).

11. Procédé selon la revendication 9, dans lequel le temps de réaction se monte à entre 2 et 20 heures, lorsque X₁ représente un atome d'halogène dans la formule (2).

12. Procédé selon l'une des revendications 1 à 11, dans lequel la température de réaction se monte à entre 80 et 95°C.

13. Procédé selon l'une des revendications 1 à 12, dans lequel on utilise comme base un alkylate d'alkyle ou une trialkylamine.
